# EUROPEAN PATENT SPECIFICATION

(11) **EP 0 998 444 B1**
(45) Date of publication and mention of the grant of the patent: **05.03.2003**
(21) Application number: 98932567.5
(22) Date of filing: 17.07.1998
(51) Int. Cl.: C07C 69/013, C07C 67/54, C07C 69/14, C07C 29/20, C07C 35/08, A61K 7/46

(54) **PROCESS FOR THE PREPARATION OF 4-TERT.-BUTYLCYCLOHEXYL ACETATE**
VERFAHREN ZUR HERSTELLUNG VON 4-TERT.-BUTYLCYCLOHEXYLACETAT
PROCEDE POUR LA PREPARATION DE 4-TERT.-BUTYLCYCLOHEXYL ACETATE

(30) Priority: 17.07.1997 JP 19266397; 25.07.1997 JP 19994097
(43) Date of publication of application: 10.05.2000
(73) Proprietor: SUMITOMO CHEMICAL COMPANY LIMITED, Osaka-shi, Osaka 541-0041 (JP); Toyotama International Co., Ltd., Tokyo 103-0014 (JP)
(72) Inventor: SEKIGUCHI, Masahito, Niihama-shi Ehime 792-0009 (JP); TANAKA, Shin, Niihama-shi Ehime 792-0009 (JP); TSUKASA, Hidetaka, Atsugi-shi Kanagawa 243-0003 (JP)
(74) Representative: VOSSIUS & PARTNER
(86) International application number: JP9803219
(87) International publication number: WO99003815

(56) References cited:
- US-A- 2 840 599

## Description

The present invention relates to 4-tert.-butylcyclohexyl acetate containing at least 90 % of a cis-isomer, and a perfume composition comprising the same.

### BACKGROUND ART

4-tert.-Butylcyclohexyl acetate (hereinafter referred to as "BCA") has attractive woody and fruity fragrance, and is used as a perfume in soaps, detergents, shampoos.

BCA has two isomers, that is, a cis-isomer and a trans-isomer (hereinafter, the cis-isomeric BCA being referred to as "CBCA", while the trans-isomeric BCA being referred to as "TBCA"), and thus BCA is commercially distributed in the form of the mixture of two isomers. In the general commercial product of BCA, the ratio of CBCA to TBCA is about 3:7. The high quality product of BCA in which CBCA is enriched up to 70 % is available. BCA containing CBCA in a higher content is still desired, since CBCA has more preferable fragrance than TBCA.

The present inventors noticed that it is desirable to use 4-tert.-butylcyclohexanol having a high cis-isomer content to obtain BCA having a high CBCA content, and carried out investigations for the easy preparation of 4-tert.-butylcyclohexanol having a high cis-isomer content on a commercial scale. As the result of the investigations, it was found that 4-tert.-butylcyclohexanol having a high its cis-isomer content can be prepared by hydrogenating 4-tert.-butylphenol in the presence of a rhodium catalyst and a solvent, and also in the presence of a specific acid (see JP-A-9-87218).

US-A 2,840,599 discloses BCA in which the CBCA content is increased to 84%.

The problem to be solved by the invention is to provide BCA having a better fragrance than conventional BCA. The solution is provided by the process as defined in claim 1 or 2.

In particular, the present invention provides a process for the preparation of the above-mentioned BCA comprising the steps of:
i) hydrogenating 4-tert.-butylphenol in the presence of a rhodium catalyst and a solvent and in the presence of at least one compound selected from the group consisting of hydrogen chloride, (anhydrous) sulfuric acid, perchloric acid, hydrogen bromide and bromine to obtain 4-tert.-butylcyclohexanol,
ii) acetylating the obtained 4-tert.-butylcyclohexanol to obtain BCA, and
iii) rectifying the BCA at a temperature of 140°C or lower in the presence of an alkali.

(Hereinafter, the term "(anhydrous) sulfuric acid" means both anhydrous sulfuric acid and sulfuric acid.)

BCA as obtained by the present process have attractive woody and fruity fragrance, and is preferably used as, for example, a perfume.

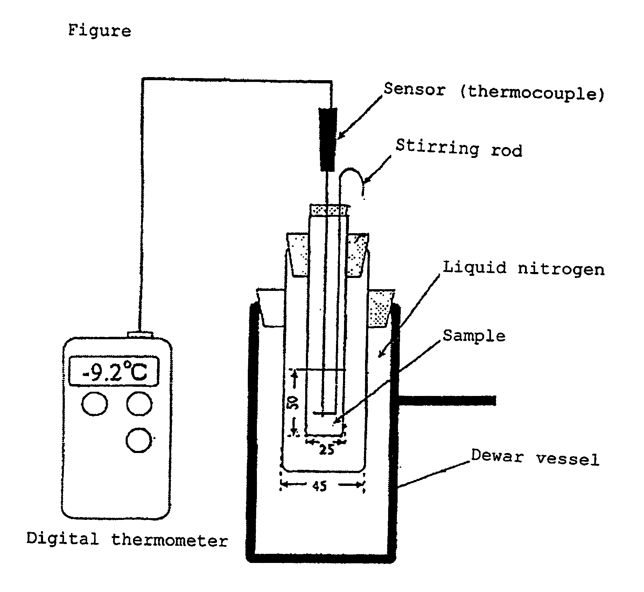

### BRIEF DESCRIPTION OF THE DRAWING

The Figure schematically shows an apparatus for the measurement of a freezing point, which is used in Examples.

### DETAILED DESCRIPTION OF THE INVENTION

4-tert.-Butylcyclohexyl acetate (BCA) of the present invention contains at least 90 % of CBCA and 50 ppm or less of acetic acid, and has a freezing point of -35°C or higher.

When the content of acetic acid in BCA exceeds about 50 ppm, desirable perfume cannot be obtained even if the content of CBCA in BCA is sufficiently high.

Herein, "%" is "wt. %" based on the whole weight of obtained BOA, unless otherwise indicated.

The content of CBCA in BCA of the present invention is preferably at least 95 %, more,preferably at least 99 %. The upper limit of the CBCA content is not limited, and may be 100 %.

The content of acetic acid in BCA of the present invention is preferably 30 ppm or less, more preferably 20 ppm or less. The lower limit of the acetic acid content is not limited, and may be 0 ppm.

The freezing point of BCA of the present invention is preferably -20°C or higher, more preferably -10°C or higher. The 'upper limit of the freezing point is not limited.

The contents of impurities contained in BCA of the present invention preferably do not exceed the following respective values.

The content of 4-tert.-butylcyclohexanol is preferably 500 ppm or less, more preferably 200 ppm or less, in particular 100 ppm or less.

The content of 4-tert.-butylcyclohexene is preferably 500 ppm or less, more preferably 200 ppm or less, in particular 100 ppm or less.

The content of cis-3-tert.-butylcyclohexyl acetate is preferably about 3,000 ppm or less, more preferably 1,000 ppm or less, in particular 500 ppm or less.

When the contents of the impurities are within the above limits, BCA of the present invention has very preferable perfume.

Furthermore, the content of p-tert.-butylphenol in BCA of the present invention is preferably 500 ppm or less, more preferably about 200 ppm or less, in particular 20 ppm or less.

The content of water is preferably 1,000 ppm or less, more preferably 500 ppm or less, in particular 250 ppm or less.

When the content of p-tert.-butylphenol or water exceeds the above limit, the stability of BCA may deteriorate over time, for example, BCA may be denatured and colored.

BCA of the present invention can be prepared by any method insofar as the content of CBCA is at least 90 %, the content of acetic acid is 50 ppm or less, and the freezing point is -35°C or higher.

An exemplary method comprises the steps of i) hydrogenating 4-tert.-butylphenol in the presence of a rhodium catalyst and a solvent and also in the presence of at least one compound selected from the group consisting of hydrogen chloride, (anhydrous) sulfuric acid, perchloric acid, hydrogen bromide and bromine to obtain 4-tert.-butylcyclohexanol, ii) acetylating the obtained 4-tert.-butylcyclohexanol to obtain BCA, and iii) rectifying the BCA at a temperature of 140°C or lower in the presence of an alkali.

Examples of the rhodium catalyst include metal rhodium and rhodium compounds in which the valence of rhodium is from 0 to 6, such as metal rhodium, rhodium chloride or rhodium oxide.

Such metal rhodium or rhodium compounds are preferably used in the form of a supported catalyst comprising the metal rhodium or rhodium compounds supported on a carrier such as activated carbon, SiO₂ or Al₂O₃. In particular, metal rhodium having the valence of 0 supported on activated carbon is preferably used. In the case of a supported catalyst, the supported percentage of metal rhodium on a carrier is usually from 1 to 10 wt. %, preferably from 3 to 5 wt. %.

After the reaction, the rhodium catalyst may be recovered from the reaction mixture by any conventional method such as filtration, decantation or centrifugation. The recovered rhodium catalyst may be recycled.

The amount of the rhodium catalyst is from 0.01 to 1 wt. % in terms of elemental rhodium, based on the weight of 4-tert.-butylphenol as a raw material. When the rhodium catalyst supported on a carrier is used, the amount of the catalyst including the carrier (dry weight) depends on the supported amount of rhodium and is usually from 0.1 to 50 wt. % based on the weight of 4-tert.-butylphenol as a raw material. The selectivity of the cis-isomer of 4-tert.-butylcyclohexanol increases as the amount of the used catalyst increases. The preferable amount of the supported catalyst is from 0.5 to 10 wt. % from the viewpoint of the operability of a filtration process for recovering the catalyst and the cost.

Any solvent can be used unless the solvent has any adverse effect on the reaction. Solvents which are in the liquid state at room temperature (about 25°C) are preferable because of easy handling. Preferred examples of solvents include alkanes having 5 to 10 carbon atoms, ethers having 4 to 10 carbon atoms or alcohols having 1 to 6 carbon atoms. Specific examples of the solvents are acyclic alkanes (e.g. pentane, hexane, heptane), cyclic alkanes (e.g. cyclohexane), acyclic ethers (e.g. diethyl ether), cyclic ethers (e.g. tetrahydrofuran, dioxane), and alcohols (e.g. methanol, ethanol, propanol, isopropanol, butanol, isobutanol, cyclohexanol, cyclopentanol, ). Among them, cyclohexane and isopropanol are preferred, and isopropanol is particularly preferred.

The amount of a solvent is usually from 0.2 to 20 times, preferably from 0.4 to 5 times the weight of 4-tert.-butylphenol.

The above-mentioned hydrogenation reaction is carried out in the presence of at least one compound selected from the group consisting of hydrogen chloride, (anhydrous) sulfuric acid, perchloric acid, hydrogen bromide and bromine.

Such a compound may be present in the reaction system in any form. For example, when hydrogen chloride is used, it may be blown in the reaction system in the form of hydrogen chloride gas, or added to the reaction system in the form of hydrochloric acid. Hydrochloric acid may be generated to be present in the reaction system. For example, AlCl₃ or TiCi₄ and water are charged in the reaction system to generate hydrochloric acid. Alternatively, a catalyst which generates hydrogen chloride during the reaction, such as rhodium chloride, may be used for supplying hydrogen chloride.

When (anhydrous) sulfuric acid is used, SO₃ gas may be blown in the reaction system, or an aqueous sulfuric acid solution may be added to the reaction system.

When perchloric acid is'used, its aqueous solution may be used.

When hydrogen bromide is used, it may be supplied in the same manner as in the case of hydrogen chloride. That is, it may be blown in the reaction system in the form of hydrogen bromide gas or added to the reaction system in the form of hydrobromic acid. Alternatively, it may be generated in the reaction system by charging AlBr₃ or TiBr₄ or bromine and water in the reaction system.

When bromine is used, it may be charged in the reaction system in the form of a liquid or a gas.

In the hydrogenation reaction, the order of the addition of the raw material, rhodium catalyst, solvent, and hydrogen chloride, (anhydrous) sulfuric acid, perchloric acid, hydrogen bromide or bromine is not limited.

The amount of at least one compound selected from the group consisting of hydrogen chloride, (anhydrous) sulfuric acid, perchloric acid, hydrogen bromide and bromine is from 0.01 to 100 moles, preferably from 0.05 to 10 moles, more preferably from 0.1 to 10 moles, in particular from 1.4 to 10 moles, per one mole of elemental rhodium in the rhodium catalyst.

The above-mentioned hydrogenation of 4-tert.-butylphenol may be carried out in the stream of hydrogen, or under the pressurized atmosphere of hydrogen. Preferably, the hydrogenation is carried out under a pressurized atmosphere of hydrogen from the viewpoint of a reaction rate. In this case, a pressure-resistant reactor is used.

When the hydrogenation is carried out under the pressurized atmosphere of hydrogen, the partial pressure of hydrogen is usually at least about 1.5 x 10⁵ Pa. Preferably, the partial pressure of hydrogen is from 3 x 10⁵ to 2 x 10⁶ Pa, more preferably from 5 x 10⁵ to 1.5 x 10⁶ Pa from the viewpoint of the reaction rate, the selectivity to a cis-isomer, and the pressure-resistance of production facilities.

The reaction temperature in the hydrogenation reaction is preferably at least 20°C from the viewpoint of the reaction rate and the cis-isomer selectivity of 4-tert.-butylcyclohexanol, and preferably not higher than 100°C from the viewpoint of the cis-isomer selectivity. More preferably, the reaction temperature is from 40 to 80°C from the viewpoint of the reaction rate and cis-isomer selectivity.

The hydrogenation reaction can be carried out by a batch process or a continuous process.

The end point of the hydrogenation reaction can be determined by a conventional method. For example, the reaction mixture is analyzed and a time when the conversion of 4-tert.-butylphenol reaches about 100 % is used as the end point, or a time when no substantial decrease of the hydrogen partial pressure is observed is used as the end point.

Thus obtained 4-tert.-butylcyclohexanol has a high cis-isomer content, and therefore the acetylation of such 4-tert.-butylcyclohexanol provides BCA having a high cis-isomer content.

The acetylation reaction may be carried out continuously from the previous hydrogenation of 4-tert.-butylphenol. Alternatively, 4-tert.-butylcyclohexanol may be once isolated and recovered from the reaction mixture obtained by the previous hydrogenation, and then acetylated in a separate step.

The acetylation of 4-tert.-butylcyclohexanol is usually carried out using any conventional acetylating agent such as acetic anhydride, acetic acid or acetyl chloride.

The amount of an acetylating agent is usually from 1 to 5 moles, preferably from 1 to 1.5 moles, per one mole of 4-tert.-butylcyclohexanol.

The reaction temperature for the acetylation is usually from room temperature (25°C) to 150°C, preferably from room temperature to 130°C.

The end point of the acetylation reaction can be determined by a conventional method. For example, the reaction mixture is analyzed and a time when the conversion of 4-tert.-butylcyclohexanol reaches 100 % is used as the end point of the reaction.

A solvent is not always used in the acetylation reaction. Solvents which have no influence on the acetylation reaction may be used. Preferably, solvents which are in the liquid state at room temperature are used because of good handling properties. Examples of such solvents include hydrocarbons having 5 to 10 carbon atoms or ether having 4 to 10 carbon atoms. Specific examples of the solvents are saturated hydrocarbons (e.g. pentane, hexane, heptane, cyclohexane), aromatic hydrocarbons (e.g. toluene), acyclic ethers (e.g. diethyl ether), or cyclic ethers (e.g. tetrahydrofuran).

Among them, toluene and cyclohexane are preferable.

The acetylation reaction may be carried out in the presence of a catalyst in addition to an acetylating agent.

The kind of a catalyst to be used together with an acetylating agent depends on the kind of the acetylating agent. When acetic anhydride is used as an acetylating agent, a catalyst may be sulfuric acid, hydrochloric acid, p-toluenesulfonic acid, zinc chloride, sodium acetate or pyridine. When acetic acid is used as an acetylating agent, a catalyst may be sulfuric acids. Among the catalysts, sulfuric acid is preferably used, since it is less expensive than other catalysts.

The amount of a catalyst is usually from 0.01 to 5 mole %, preferably from 0.1 to 2 mole %, based on 4-tert.-butylcyclohexanol. When the amount of the catalyst is excessive, 4-tert.-butylcyclohexanol tends to be dehydrated, undesirably.

When acetic acid is used as an acetylating agent, the acetylation reaction is preferably carried out while removing generated water. Water may be removed, for example, by evaporating water azeotropically with a solvent which can form an azeotropic mixture with water under refluxing conditions under atmospheric pressure or reduced pressure, or by adding a dehydrating agent such as silica gel to the reaction mixture.

When acetyl chloride is used as an acetylating agent, the reaction is preferably carried out while removing by-produced hydrogen chloride from the viewpoint of safety. For removing hydrogen chloride, an inorganic base such as sodium hydroxide, potassium carbonate, or an organic base such as pyridine can be added to the reaction system.

Acetic anhydride is preferably used as an acetylating agent from the viewpoint of the conversion of 4-tert.-butylcyclohexanol in the acetylation reaction.

It is difficult to isolate and purify the BCA obtained from 4-tert.-butylcyclohexanol since 4-tert.-butylcyclohexanol has a boiling point close to that of BCA. Thus, the conversion of 4-tert.-butylcyclohexanol is preferably as close to 100 % as possible. To this end, it is preferable to acetylate 4-tert.-butylcyclohexanol using, for example, acetic acid or acetyl chloride as an acetylating agent until the conversion of 4-tert.-butylcyclohexanol becomes about 90 % or larger, and then acetylate remaining 4-tert.-butylcyclohexanol with acetic anhydride in at least an equimolar amount to remaining 4-tert.-butylcyclohexanol so that 4-tert.-butylcyclohexanol is consumed completely.

The reaction mixture obtained from the acetylation reaction is optionally washed, and then rectified to obtain BCA having more desirable fragrance.

The reaction mixture from the acetylation reaction may be washed with an aqueous alkaline solution. Examples of the aqueous alkaline solution are aqueous solutions of sodium hydroxide, potassium hydroxide, sodium carbonate, sodium hydrogencarbonate (sodium bicarbonate) or potassium hydrogencarbonate. The concentration of such aqueous alkaline solutions is usually from 1 to 20 wt. %. Among them, an about 5 wt. % aqueous solution of sodium hydrogencarbonate or sodium carbonate is preferable.

The washing temperature is usually 80°C or lower, preferably 50°C or lower.

When an organic base is used as a catalyst for the acetylation reaction, the reaction mixture from the acetylation reaction is preferably washed with a weakly acidic aqueous solution such as diluted hydrochloric acid prior to the washing with the aqueous alkaline solution. When a solvent is used in the acetylation reaction, the reaction mixture is preferably concentrated just after the acetylation reaction or after the washing with the alkaline solution.

The rectification is preferably carried out in the presence of an alkali for preventing the liberation of acetic acid from BCA. A bottom temperature during the rectification is preferably 140°C or lower, more preferably from 100 to 140°C.

Examples of the alkali to be present are inorganic alkalis such as sodium hydroxide, potassium hydroxide, calcium hydroxide, sodium carbonate, potassium carbonate or calcium carbonates. Among them, sodium carbonate is preferable.

The amount of an alkali to be used is usually from 0.001 to 10 wt. %, preferably from 0.005 to 3 wt. %, more preferably from 0.01 to 0.5 wt. %, in particular from 0.02 to 0.1 wt. %, based on the weight of a liquid to be rectified in a sill pot.

The rectification is usually carried out using a distillation column having the number of plates of from 10 to 50 under a nitrogen atmosphere while maintaining the column head pressure at reduced pressure of about 10 Torr at a reflux ratio of from 3 to 40. It is possible to use a rectification column having the large number of plates or to repeat the rectification twice or more, for increasing the content of CBCA in the product.

The above-described method can provide BCA of the present invention which has a CBCA content of at least 90 %, an acetic acid content of 50 ppm or less, and a freezing point of -35°c or higher. Since such BCA has very attractive woody and fruity fragrance, it is used as a perfume, and also used in any fields which require such fragrance. For example, BCA of the present invention is mixed with conventional natural perfumes and/or synthetic perfumes, and preferably used as a perfume composition.

Examples of perfumes which can be mixed with BCA of the present invention include aldehydes (e.g. citral, hydroxycitronellal, α-hexylcinnamaldehyde, methylnonylacetoaldehyde, phenylacetoaldehyde, anisaldehyde, vanillin), alcohols (e.g. farnesol, geraniol, linalool, nerol, phenylethyl alcohol, rhodinol, cinnamic alcohol, cis-3-hexenol, menthol, α-tocopherol), ketones (e.g. berbenone, geranylacetone, α-ionone, β-ionone, isomethyl-α-ionone, allylionone), lactones (e.g. γ-undecalactone, σ-decalactone), esters (e.g. benzyl acetate, geranyl acetate, vetiveryl acetate, cis-3-hexenyl acetate, linalyl acetate, methyl dihydrojasmonate, styrallyl acetate, allylphenoxy acetate, cinnamyl propionate, citronelyl acetate, decyl acetate, benzyl salicylate, cis-3-hexenyl isobutyrate), indole, eugenol, anethole, p-menthan-8-thiol-3-on or methyleugenol. At least one of these fragrances can be combined with BCA of the present invention, and used as a perfume composition.

The mixing ratio of BCA and the other perfume or perfumes in the perfume composition depends on the properties and amounts of other components, and the desired fragrance of a perfume composition. In general, the content of BCA in the perfume composition is from about 0.01 to 50 wt. % of the whole perfume composition. However, the content of BCA may be lower or higher than this range in some applications.

The perfume compositions can be used in a wide variety of applications. Since these compositions have fruity and gorgeous fragrance, they can be used in the application fields of fragrance such as detergents, soaps, shampoos or cosmetics.

BCA as obtained by the present process has more attractive woody and fruity fragrance with stronger and more gorgeous aroma than conventional BCA, and the perfume compositions comprising such BCA have the same fragrance. BCA as obtained by the present process can be used as a perfume used in soaps, detergents and shampoos, and also used in a wide variety of applications such as cosmetics.

### EXAMPLES

The present invention will be illustrated by the following Examples, which will not limit the scope of the invention in any way.

In the following Examples, the freezing point of BCA was measured as follows:

As shown in the Figure, a sample of BCA is filled to a height of 5 cm of a glass tube having an inner diameter of 2.5 cm. The glass tube is set in another glass tube having an inner diameter of 4.5 cm. Then, the glass tubes are dipped in liquid nitrogen contained in a Dewar vessel. A stirring rod was moved vertically at a rate of about 100 times/min., and the change of internal temperature is monitored with a digital thermometer.

The fragrance test was carried out as follows:

The fragrance of a sample perfume is smelled by five female panels whose ages are from twenties to forties. The fragrance is ranked by the marks 5, 4, 3, 2 and 1, with the best fragrance being ranked "5", while the poorest fragrance being ranked "1". Then, the marks of the five panels are summed for each perfume, and the perfume having the highest total score is marked "A", and others are marked B, C, D and E in the order of the total scores.

The abbreviations used in the Examples are as follows:
CBCA: cis-4-tert.-Butylcyclohexyl acetate.
TBCA: trans-4-tert.-Butylcyclohexyl acetate.
BCHE: 4-tert.-Butyl-1-cyclohexene.
BCL: 4-tert.-Butylcyclohexanol.
BPN: p-tert.-Butylphenol.
MA2: cis-3-tert.-Butylcyclohexyl acetate.

### Example 1

p-tert.-Butylphenol (330.1 kg, 2197 mole), 5%Rh/C (5 wt. % of metal rhodium supported on activated carbon carrier) (3.3 kg, dry weight), isopropanol (660.2 kg) and 36 % hydrochloric acid (0.225 kg) were charged in an autoclave. The internal atmosphere was replaced with nitrogen by pressurizing the internal space with nitrogen up to 3 x 10⁵ Pa and evacuating nitrogen three times, and then with hydrogen by pressurizing the internal space with hydrogen up to 3 x 10⁵ Pa and evacuating hydrogen three times. After that, hydrogen was injected in the autoclave up to 6 x 10⁵ Pa, and the mixture in the autoclave was stirred at 50°C for 15 hours to carry out the hydrogenation reaction.

The autoclave was cooled, and the internal atmosphere was replaced with nitrogen by the same procedures as described above, and the reaction mixture was analyzed. The yield of 4-tert.-butylcyclohexanol was 97.4 %, and a cis/trans ratio was 91.7:8.3.

After filtrating off the catalyst from the reaction mixture, the reaction mixture was concentrated to obtain 4-tert.-butylcyclohexanol having a cis/trans ratio of 91.5:8.5. Then, toluene (355 kg) and 98 % sulfuric acid (2.07 kg) were added to the 4-tert.-butylcyclohexanol. The internal temperature of the autoclave was raised to 92.5°C under reduced pressure of 400 Torr (53.4 kPa), and acetic acid (161.3 kg, 2685 moles) was dropwise added over 1 hour while refluxing, and then the same temperature was maintained for 4 hours. Furthermore, acetic anhydride (32.3 kg, 316.3 moles) was added, and the reaction mixture was refluxed at an internal temperature of 103°C under pressure of 400 Torr (53.4 kPa) for 2 hours to further carry out the acetylation reaction. Then, the reaction mixture was analyzed. The yield of 4-tert.-butylcyclohexyl acetate was 95.8 % (based on 4-tert.-butylcyclohexanol), and a cis/trans ratio was 91.2:8.8.

The reaction mixture was concentrated, and washed with a 5 % aqueous solution of sodium hydrogencarbonate (382 kg) once to obtain an oil layer (400.7 kg).

The procedures of the hydrogenation reaction, acetylation reaction and washing with the aqueous solution of sodium hydrogencarbonate were repeated three times in the same way as described above. The oil layers from the four batches were combined (total weight, 1744 kg), and washed with a 5 % aqueous solution of sodium hydrogencarbonate. One third of obtained crude 4-tert.-butylcyclohexyl acetate was charged in a rectification column having the number of plates of 13, and rectified in the presence of sodium carbonate (150 g), under nitrogen atmosphere while maintaining the column head pressure at 10 Torr (1.3 kPa) . During the rectification, the bottom temperature was maintained at 140°C, and fractions having lower and higher boiling points than that of cis-4-tert.-butylcyclohexyl acetate were removed, so that the cis-isomer content was increased to 97 % or higher. Each half of the rest of crude 4-tert.-butylcyclohexyl acetate was rectified in the same manner as described above, respectively. All the 4-tert.-butylcyclohexyl acetate fractions obtained in the three rectification processes were combined and again rectified in the presence of sodium carbonate, and thus 4-tert.-butylcyclohexyl acetate (1050 kg) having a cis-isomer content of 99 % or higher was obtained.

The composition of the obtained 4-tert.-butylcyclohexyl acetate was analyzed. The product contained 99 % of cis-4-tert.-butylcyclohexyl acetate, 1 % of trans-4-tert.-butylcyclohexyl acetate, 20 ppm of acetic acid, 316 ppm of cis-3-tert.-butylcyclohexyl acetate, 17 ppm of 4-tert.-butylphenol, 43 ppm of 4-tert.-butylcyclohexanol, 89 ppm of 4-tert.-butyl-1-cyclohexene and 470 ppm of water. The freezing point was -9°C.

The results of the above analysis and the results of the fragrance test of obtained 4-tert.-butylcyclohexyl acetate are summarized in Table 1.

### Examples 2-3 and Comparative Example 1

4-tert.-Butylcyclohexyl acetate obtained in Example 1 and trans-4-tert.-butylcyclohexyl acetate (purity of 99 %) were mixed in suitable amounts to prepare three samples having a cis-isomer content of 95 % (Example 2), 90 % (Example 3), and 85 % (Comparative Example 1), respectively.

The used trans-4-tert.-butylcyclohexyl acetate (purity of 99 %) was obtained by recovering the fraction having the higher boiling point during the rectification of 4-tert.-butylcyclohexyl acetate in Example 1, and again rectifying the recovered trans-isomer.

The three samples were analyzed. The results of the analysis and the results of the fragrance test of the three samples are summarized in Table 1.

### Comparative Example 2

An oil layer (596.5 g) was obtained in the same manner as in Example 1 after the washing with a 5 % aqueous solution of sodium hydrogencarbonate and prior to the rectification. Then, the oil layer was washed with water (149.1 g). The same procedures as above were repeated, and the oil layers after washing with water (total amount of 750 g) were combined and rectified using a rectification column having the number of plates of about 20, under a nitrogen atmosphere while maintaining the column head pressure at 10 Torr (1.3 kPa) at a reflux ratio of 15. During the rectification, the bottom temperature was 140°C. In the first rectification, fractions having lower and higher boiling points than that of cis-4-tert.-butylcyclohexyl acetate were discarded, so that the cis-isomer content was increased to 98 % or higher. The obtained 4-tert.-butylcyclohexyl acetate was again rectified to increase the cis-isomer content to 99 % or higher. The obtained 4-tert.-butylcyclohexyl acetate was analyzed. The results of the analysis and the results of the fragrance test are summarized in Table 1.

**Table 1**

| | Example No. | | | | |
|---|---|---|---|---|---|
| | 1 | 2 | 3 | Comp. 1 | Comp. 2 |
| CBCA (%) | 99 | 95 | 90 | 85 | 99.9 |
| TBCA (%) | 1 | 5 | 10 | 15 | <0.1 |
| Acetic acid (ppm) | 20 | 20 | 20 | 20 | 94 |
| Water (ppm) | 470 | 470 | 470 | 470 | Not measured |
| BCHE (ppm) | 89 | 92 | 94 | 75 | 266 |
| BCL (ppm) | 43 | 43 | 43 | 43 | 22 |
| BPN (ppm) | 17 | 17 | 17 | 17 | Not detected |
| MA2 (ppm) | 316 | 236 | 237 | 247 | 53 |
| Freezing point (°C) | -9 | -17 | -33 | Unmesurable | Not measured |
| Result of fragrance test | A | B | C | D | E |

### Example 4 and Comparative Examples 3-4

Rose type perfume compositions A, B and C for soaps were respectively prepared by mixing the components shown in Table 2. The unit of each amount in Table 2 is parts by weight.

BCA having a cis-isomer content of 70 % and BCA having a cis-isomer content of 30 % were both commercial products. BCA having a cis-isomer content of 99 % was prepared in the same manner as in Example 1.

**Table 2**

| | Composition | | |
|---|---|---|---|
| | A (Ex. 4) | B (C. E. 3) | C (C. E. 4) |
| Ylang-ylanq oil | 15 | 15 | 15 |
| Base 20-H^{[1]} | 20 | 20 | 20 |
| Galaxolide®^{[2]} | 20 | 20 | 20 |
| Limonene | 25 | 25 | 25 |
| Santalex®^{[3]} | 25 | 25 | 25 |
| Methyl cedrenyl ketone | 25 | 25 | 25 |
| Dimethylbenzylcarbinyl acetate | 30 | 30 | 30 |
| α-Hexylcinnamic aldehyde | 40 | 40 | 40 |
| Benzyl acetate | 60 | 60 | 60 |
| Muguet base^{[1]} | 65 | 65 | 65 |
| BCA (cis-isomer, 99 %) | 65 | 0 | 0 |
| BCA (cis-isomer, 70 %) | 0 | 65 | 0 |
| BCA (cis-isomer, 30 %) | 0 | 0 | 65 |
| Rose base^{[1]} | 80 | 80 | 80 |
| Terpineol | 80 | 80 | 80 |
| Rose P | 100 | 100 | 100 |
| Base 100-P^{[1]} | 100 | 100 | 100 |
| Linalool | 110 | 110 | 110 |
| Dipropylene glycol | 140 | 140 | 140 |
| Total | 1000 | 1000 | 1000 |

| | | | |
|---|---|---|---|
| 1. The product produced by Toyotama International Co., Ltd. | | | |
| 2. The product produced by IFF Co., Ltd. | | | |
| 3. The product produced by Takasago International Co., Ltd. | | | |

Composition A had very attractive, gorgeous and fruity fragrance, while Composition B had moderate fragrance which was less attractive than the fragrance of Composition A. The fragrance of Composition C was inferior to that of Compositions A and B.

### Example 5 and Comparative Examples 5-6

Floral type perfume compositions D, E and F for shampoos were respectively prepared by mixing the components shown in Table 3. The unit of each amount in Table 3 is parts by weight.

BCA having a cis-isomer content of 70 %, BCA having a cis-isomer content of 30 % and BCA having a cis-isomer content of 99 % were the same as those used in Example 4, Comparative Example 3 and Comparative Example 4, respectively.

**Table 3**

| | Composition | | |
|---|---|---|---|
| | D (Ex. 5) | E (C. E. 5) | F (C. E. 6) |
| Diphenyl oxide | 10 | 10 | 10 |
| Limonene | 15 | 15 | 15 |
| Galbanadol®^{[4]} | 20 | 20 | 20 |
| Patchouli oil | 20 | 20 | 20 |
| Coumarin | 20 | 20 | 20 |
| Amber base^{[1]} | 30 | 30 | 30 |
| Bergamot oil | 40 | 40 | 40 |
| Jasminal | 50 | 50 | 50 |
| Tricyclodecenyl propionate | 50 | 50 | 50 |
| Dipropylene glycol | 50 | 50 | 50 |
| Benzyl acetate | 80 | 80 | 80 |
| Floral base^{[1]} | 100 | 100 | 100 |
| Nopyl acetate | 100 | 100 | 100 |
| BCA (cis-isomer, 99 %) | 200 | 0 | 0 |
| BCA (cis-isomer, 70 %) | 0 | 200 | 0 |
| BCA (cis-isomer, 30 %) | 0 | 0 | 200 |
| Muguet base^{[1]} | 215 | 215 | 215 |
| Total | 1000 | 1000 | 1000 |

| | | | |
|---|---|---|---|
| 1. The product produced by Toyotama International Co., Ltd. | | | |
| 4. The product produced by ROBERTET Co., Ltd. | | | |

Composition D had very attractive, gorgeous and fruity fragrance, while Composition E had moderate fragrance which was less attractive than the fragrance of Composition D. The fragrance of Composition F was inferior to that of Compositions D and E.

## Claims

1. A process for the preparation of 4-tert.-butylcyclohexyl acetate having a cis-isomer content of at least 90 %, containing 50 ppm or less of acetic acid, and having a freezing point of -35°C or higher, comprising the step of rectifying 4-tert.-butylcyclohexyl acetate at a temperature of 140°C or lower in the presence of an alkali.

2. A process for the preparation of 4-tert.-butylcyclohexyl acetate having a cis-isomer content of at least 90 %, containing 50 ppm or less of acetic acid, and having a freezing point of -35°C or higher according to claim 1, comprising the steps of:
i) hydrogenating 4-tert.-butylphenol in the presence of a rhodium catalyst and a solvent and in the presence of at least one compound selected from the group consisting of hydrogen chloride, (anhydrous) sulfuric acid, perchloric acid, hydrogen bromide and bromine to obtain 4-tert.-butylcyclohexanol,
ii) acetylating the obtained 4-tert.-butylcyclohexanol to obtain 4-tert.-butylcyclohexyl acetate, and
iii) rectifying the 4-tert.-butylcyclohexyl acetate at a temperature of 140°C or lower in the presence of an alkali.

## Patentansprüche

1. Verfahren zur Herstellung von 4-tert.-Butylcyclohexylacetat mit einem cis-Isomer-Gehalt von mindestens 90 %, enthaltend 50 ppm oder weniger Essigsäure und mit einem Gefrierpunkt von -35°C oder höher, umfassend den Schritt des Rektifizierens des 4-tert.-Butylcyclohexylacetats bei einer Temperatur von 140°C oder niedriger in Anwesenheit eines Alkali.

2. Verfahren zur Herstellung von 4-tert.-Butylcyclohexylacetat mit einem cis-Isomer-Gehalt von mindestens 90 %, enthaltend 50 ppm oder weniger Essigsäure und mit einem Gefrierpunkt von -35°C oder mehr nach Anspruch 1, umfassend die Schritte:
(i) Hydrieren von 4-tert.-Butylphenol in Anwesenheit eines Rhodiumkatalysators und eines Lösungsmittels und in Anwesenheit von mindestens einer Verbindung ausgewählt aus der Gruppe bestehend aus Chlorwasserstoff, (wasserfreier) Schwefelsäure, Perchlorsäure, Bromwasserstoff und Brom, um 4-tert.-Butylcyclohexanol zu erhalten,
(ii) Acetylieren des erhaltenen 4-tert.-Butylcyclohexanols, um 4-tert.-Butylcyclohexylacetat zu erhalten, und
(iii) Rektifizieren des 4-tert.-Butylcyclohexylacetats bei einer Temperatur von 140°C oder niedriger in Anwesenheit eines Alkali.

## Revendications

1. Procédé pour la préparation d'acétate de 4-tert.-butylcyclohexyle ayant une teneur en isomère cis d'au moins 90%, contenant 50 ppm ou moins d'acide acétique, et ayant un point de congélation de -35°C ou supérieur, comprenant l'étape de rectification d'acétate de 4-tert.-butylcyclohexyle à une température de 140°C ou inférieure en présence d'un alcali.

2. Procédé pour la préparation d'acétate de 4-tert.-butylcyclohexyle ayant une teneur en isomère cis d'au moins 90%, contenant 50 ppm ou moins d'acide acétique, et ayant un point de congélation de -35°C ou supérieur selon la revendication 1, comprenant les étapes :
i) d'hydrogénation de 4-tert.-butylphénol en présence d'un catalyseur de rhodium et d'un solvant et en présence d'au moins un composé choisi parmi le chlorure d'hydrogène, l'acide sulfurique (anhydre), l'acide perchlorique, le bromure d'hydrogène et le brome pour obtenir du 4-tert-butylcyclohexanol,
ii) d'acétylation du 4-tert.-butylcyclohexanol obtenu pour obtenir de l'acétate de 4-tert.-butylcyclohexyle, et
iii) de rectification de l'acétate de 4-tert.-butylcyclohexyle à une température de 140°C ou inférieure en présence d'un alcali.
